# EUROPEAN PATENT APPLICATION

(11) **EP 4 442 141 A1**
(43) Date of publication of application: **09.10.2024**
(21) Application number: 21966313.5
(22) Date of filing: 30.11.2021
(51) Int. Cl.: A24F 40/50, A24F 40/57

(54) **INHALATION DEVICE**

(71) Applicant: Japan Tobacco Inc., Tokyo 105-6927 (JP)
(72) Inventor: TEZUKA, Hiroshi, Tokyo 130-8603 (JP)
(74) Representative: Hoffmann Eitle
(86) International application number: PCT/JP2021/043807
(87) International publication number: WO 2023/100227

(57) **Abstract**

An inhalation device (100B) comprises a body (30) which is a body portion, and a panel (10) attachable to and removable from the body (30). The panel (10) comprises a sensor. The sensor comprises a light-emitting element which irradiates a user's body with light, and a light-receiving element which receives light that has passed through the user's body. The sensor outputs information relating to the light received by the light-receiving unit. The body (30) comprises a control unit that controls the operation of the inhalation device (100B) on the basis of an output from the sensor.

## Description

### TECHNICAL FIELD

The present invention relates to an inhalation device including a main body portion and a panel attachable to and detachable from the main body portion.

### BACKGROUND ART

In the related art, for example, an inhalation device that enables to inhale an aerosol containing a flavor component is known. Further, Patent Literature 1 below discloses a technique in which a controller provides health data to a user based on biological characteristics of the user detected by a biosensor provided in a main cigarette stick holding portion of an aerosol generating device.

### CITATION LIST

### PATENT LITERATURE

Patent Literature 1: Japanese Unexamined Patent Application Publication No. 2021-516540

### SUMMARY OF INVENTION

### TECHNICAL PROBLEM

However, in the related art, there is room for improvement from a viewpoint of improving convenience for the user in the inhalation device.

The present invention provides an inhalation device that further improves convenience for a user.

### SOLUTION TO PROBLEM

In the present invention, there is provided an inhalation device including a main body portion a panel attachable to and detachable from the main body portion, in which
the panel includes a sensor,
the sensor includes a light emitting element configured to irradiate a human body of a user with light and a light receiving element configured to receive the light via the human body, and outputs information related to the light received by the light receiving element, and
the main body portion includes a control unit configured to control an operation of the inhalation device based on an output of the sensor.

### ADVANTAGEOUS EFFECTS OF INVENTION

According to the present invention, it is possible to provide an inhalation device with improved convenience for a user.

### BRIEF DESCRIPTION OF DRAWINGS

FIG. 1A is a schematic diagram schematically illustrating a first configuration example of an inhalation device according to an embodiment.
FIG. 1B is a schematic diagram schematically illustrating a second configuration example of an inhalation device according to an embodiment.
FIG. 2 is a schematic diagram schematically illustrating a configuration example of a panel according to an embodiment.
FIG. 3 is an overall perspective view of the inhalation device in FIG. 1B.
FIG. 4A is a schematic external view of an example of the panel provided in the inhalation device of FIG. 1B.
FIG. 4B is a schematic external view of an example of a main body housing provided in the inhalation device of FIG. 1B.
FIG. 5A is a diagram illustrating an example of a positional relation between an operation unit and a light emitting element and a light receiving element in an inhalation device according to an embodiment.
FIG. 5B is a diagram illustrating another example of a positional relation between an operation unit and a light emitting element and a light receiving element in an inhalation device according to an embodiment.
FIG. 6 is a diagram illustrating an example of absorbance characteristics of first hemoglobin and second hemoglobin.
FIG. 7 is a diagram illustrating a first example of control based on an output of a sensor unit by a control unit of the inhalation device according to an embodiment.
FIG. 8 is a diagram illustrating a second example of the control based on the output of the sensor unit by the control unit of the inhalation device according to an embodiment.
FIG. 9 is a diagram illustrating an example of a heating profile of a heating portion in the second example.
FIG. 10 is a diagram illustrating a third example of the control based on the output of the sensor unit by the control unit of the inhalation device according to an embodiment.
FIG. 11 is an overall perspective view of an inhalation device according to a modification of an embodiment.
FIG. 12 is a diagram illustrating an example of a positional relation between an operation unit and a light emitting element and a light receiving element in the inhalation device according to the modification.

### DESCRIPTION OF EMBODIMENTS

Hereinafter, an inhalation device according to an embodiment of the present invention will be described with reference to the drawings. Hereinafter, the same or similar elements are denoted by the same or similar reference numerals, and the description thereof may be appropriately omitted or simplified. In the following description, the inhalation device is a device that generates a substance to be inhaled by the user, and includes, but is not limited to, an electronic cigarette and a nebulizer.

### «1. Configuration Example of Inhalation Device»

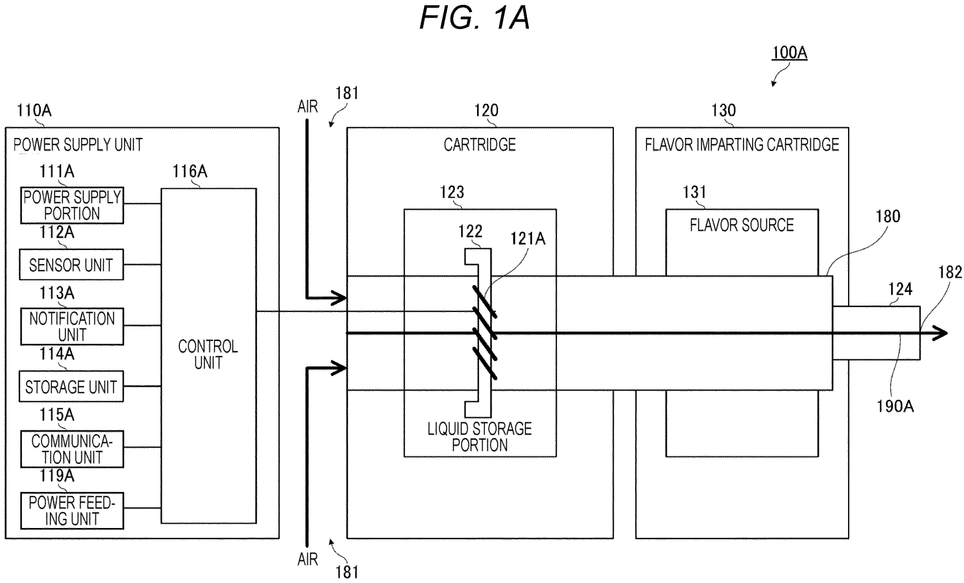

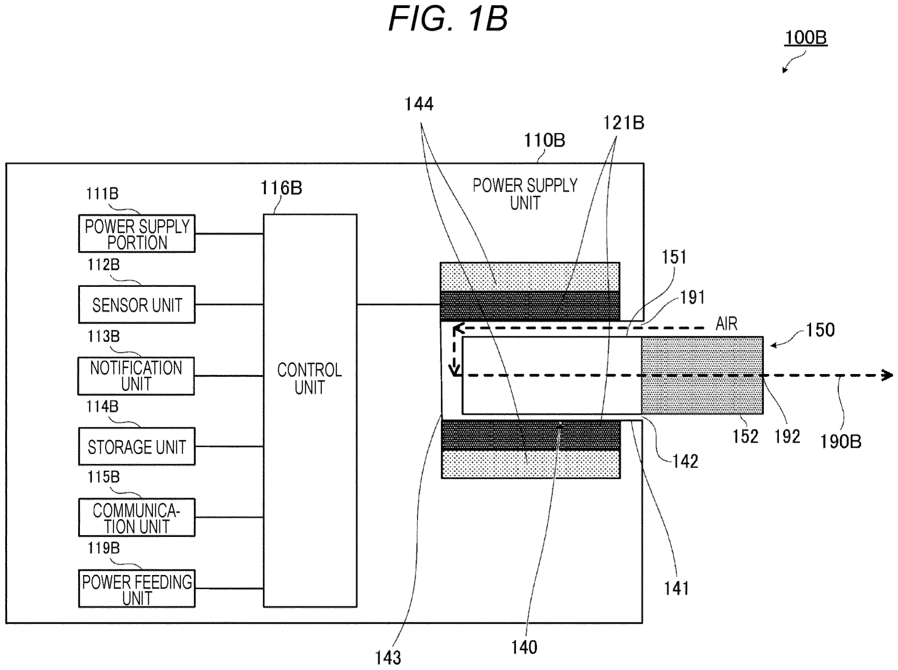

First, an inhalation device 100 (100A, 100B) according to an embodiment will be described with reference to FIGs. 1A and 1B. In the following description, it is assumed that a substance generated by the inhalation device 100 is an aerosol and an inhalation component source to be heated is an aerosol source, but the present invention is not limited thereto.

### (1.1) First Configuration Example of Inhalation Device

FIG. 1A is a schematic diagram schematically illustrating a first configuration example of the inhalation device. As illustrated in FIG. 1A, the inhalation device 100A according to this configuration example includes a power supply unit 110A, a cartridge 120, and a flavor imparting cartridge 130. The power supply unit 110A may be referred to as a main body portion of the inhalation device 100A, and includes a power supply portion 111A, a sensor unit 112A, a notification unit 113A, a storage unit 114A, a communication unit 115A, a power feeding unit 119A, and a control unit 116A. The cartridge 120 includes a heating portion 121A, a liquid guide portion 122, and a liquid storage portion 123. The flavor imparting cartridge 130 includes a flavor source 131 and a mouthpiece 124. An air flow path 180 is formed in the cartridge 120 and the flavor imparting cartridge 130. The cartridge 120 is attachable to and detachable from the power supply unit 110A, and the flavor imparting cartridge 130 is attachable to and detachable from the cartridge 120. In other words, the cartridge 120 and the flavor imparting cartridge 130 are both replaceable.

The power supply portion 111A stores electric power. The power supply portion 111A feeds the electric power to each component of the inhalation device 100A under control of the control unit 116A. The power supply portion 111A may be implemented by a rechargeable battery such as a lithium-ion secondary battery.

The sensor unit 112A obtains various types of information related to the inhalation device 100A. For example, the sensor unit 112A includes a pressure sensor such as a microphone condenser, a flow rate sensor, a temperature sensor, or the like, and obtains a value associated with inhalation by the user. As another example, the sensor unit 112A is implemented by an input device that receives input of information from the user, such as a button or a switch.

The notification unit 113A notifies the user of the various types of information. The notification unit 113A includes, for example, a light-emitting device (for example, an LED) that emits light, a display device that displays an image, a sound output device that outputs sound, or a vibrating device that vibrates.

The storage unit 114A stores various types of information (for example, data and programs) for operating the inhalation device 100A. The storage unit 114A includes, for example, a nonvolatile storage medium such as a flash memory. As an example, the storage unit 114A stores a heating profile (for example, a first heating profile Pr1 and a second heating profile Pr2 to be described later) of the heating portion 121A. Here, the heating profile is information defining a time-series transition of a target temperature of the heating portion 121A when the heating portion 121A is heated. The storage unit 114A may store data received by the power supply unit 110A from a panel 10 to be described later.

The communication unit 115A is a communication interface capable of performing communication conforming to any wired or wireless communication standard. In the case of wireless communication, for example, Wi-Fi (registered trademark), Bluetooth (registered trademark), or near field communication (NFC) may be adopted as the communication standard. In the case of wired communication, for example, communication may be performed by an external connection terminal such as a universal serial bus (USB) and a data communication cable. For example, the communication unit 115A can input and output data related to the operation of the inhalation device 100A between the power supply unit 110A and an external device.

An example of the external device which is a communication destination of the communication unit 115A (that is, the power supply unit 110A) is the panel 10 to be described later. The communication unit 115A communicates with the panel 10 by, for example, the near field communication. Accordingly, communication between the power supply unit 110A and the panel 10 is enabled with a simple configuration. In this case, the communication unit 115A may include, for example, an NFC reader and writer module and an NFC antenna.

Further, the communication unit 115A may communicate with a terminal device (for example, a smartphone) of the user or a predetermined server device (for example, a server device managed by a manufacturer of the inhalation device 100A). For example, the communication unit 115 may perform communication according to different communication standards depending on communication destinations, for example, may communicate with the panel 10 by the near field communication and communicate with the terminal device of the user by Bluetooth (registered trademark). In this way, it is possible to communicate with each communication destination according to an appropriate communication standard, and to efficiently communicate with each communication destination.

The power feeding unit 119A feeds the electric power of the power supply portion 111A to the external device of the power supply unit 110A under the control of the control unit 116A. An example of the external device to which the electric power is fed by the power feeding unit 119A is the panel 10 to be described later. The power feeding unit 119A feeds the electric power to the panel 10 by non-contact electric power transmission, for example. An example of the non-contact electric power transmission is electric power transmission by the near field communication. Accordingly, the electric power can be fed from the power supply unit 110A to the panel 10 with a simple configuration.

As described above, the communication unit 115A is configured to communicate with the panel 10 through the near field communication, and the power feeding unit 119A is configured to feed electric power to the panel 10 through the electric power transmission by the near field communication, so that the communication and electric power transmission between the power supply unit 110A and the panel 10 is efficiently enabled. Accordingly, it is possible to simplify configurations of the power supply unit 110A and the panel 10 compared with a case where the communication and the electric power transmission between the power supply unit 110A and the panel 10 are performed using different mechanisms. More specifically, in this case, the power feeding unit 119A may be implemented by the NFC reader and writer module, the NFC antenna, and the like which are the same as those of the communication unit 115A described above.

The power feeding unit 119A may feed electric power to the panel 10 not only through the electric power transmission by the near field communication but also through non-contact electric power transmission of another method. As the non-contact electric power transmission of another method, various types of non-contact electric power transmission of a non-radiation type such as an electromagnetic induction method, a magnetic field resonance method, or an electric field coupling method, or various types of non-contact electric power transmission of a radiation type such as a radio wave method or a laser method may be adopted. As a specific example of the non-contact electric power transmission of the electromagnetic induction method, Qi (registered trademark) can be used.

Further, the power supply unit 110A and the panel 10 may be connected via a physical power feeding interface, and the power feeding unit 119A may feed the electric power to the panel 10 via the power feeding interface. Examples of the physical power feeding interface include a pogo pin, a plate spring, and various connectors and cables. Further, the power feeding unit 119A may be configured to feed electric power of the power supply portion 111A to an external device (for example, the terminal device of the user) other than the panel 10. In this way, the power supply unit 110A can be utilized as a so-called "mobile battery", and convenience for the user can be improved.

The control unit 116A functions as an arithmetic processing device and a control device, and controls overall operations in the inhalation device 100A according to various programs. The control unit 116A is implemented by, for example, an electronic circuit such as a central processing unit (CPU) or a microprocessor. A specific control example by the control unit 116A will be described later.

The liquid storage portion 123 stores the aerosol source. The aerosol source is atomized and/or vaporized (hereinafter, also simply referred to as "atomized") to generate aerosol. The aerosol source is, for example, a polyhydric alcohol such as glycerin and propylene glycol, and a liquid such as water. The aerosol source may contain a flavor component derived from tobacco or non-tobacco. When the inhalation device 100A is a medical inhaler such as a nebulizer, the aerosol source may include a drug.

The liquid guide portion 122 guides and holds the aerosol source, which is a liquid stored in the liquid storage portion 123, from the liquid storage portion 123. The liquid guide portion 122 is, for example, a wick formed by twisting a fibrous material such as a glass fiber or a porous material such as a porous ceramic. When the liquid guide portion 122 is a wick, the aerosol source stored in the liquid storage portion 123 is induced by a capillary effect of the wick.

The heating portion 121A heats the aerosol source to atomize the aerosol source to generate the aerosol. In the example illustrated in FIG. 1A, the heating portion 121A is implemented by a coil and wound around the liquid guide portion 122. When the heating portion 121A generates heat, the aerosol source held by the liquid guide portion 122 is heated and atomized, and the aerosol is generated. The heating portion 121A generates heat when fed with the electric power from the power supply portion 111A.

As an example, when the sensor unit 112A detects that the user starts inhaling, that a predetermined user input operation is received, and/or that predetermined information is input, the electric power may be fed to the heating portion 121A. Then, when the sensor unit 112A detects that the user finishes inhaling, that a predetermined user input operation is received, and/or that predetermined information is input, feeding of the electric power to the heating portion 121A may be stopped.

Further, for example, the control unit 116A may set an operation mode of the inhalation device 100A to an inhalation mode, and may set a state in which electric power can be fed to the heating portion 121A in accordance with the inhalation device 100A being powered on in response to a predetermined user input operation. That is, the control unit 116A may allow electric power feeding to the heating portion 121A only when the operation mode of the inhalation device 100A is the inhalation mode. In this case, the aerosol generated by heating the aerosol source is delivered to the user only when the operation mode of the inhalation device 100A is the inhalation mode.

The flavor source 131 is a component for imparting the flavor component to the aerosol. The flavor source 131 may contain the flavor component derived from tobacco or non-tobacco.

The air flow path 180 is a flow path of air inhaled by the user. The air flow path 180 has a tubular structure having an air inflow hole 181 which is an inlet of air into the air flow path 180 and an air outflow hole 182 which is an outlet of air from the air flow path 180 as both ends. In the middle of air flow path 180, the liquid guide portion 122 is disposed on an upstream side (a side close to the air inflow hole 181), and the flavor source 131 is disposed on a downstream side (a side close to the air outflow hole 182). Air flowing in from the air inflow hole 181 along with the inhalation by the user is mixed with the aerosol generated by the heating portion 121A, passes through the flavor source 131, and is transported to the air outflow hole 182 as indicated by an arrow 190A. When a mixed fluid of the aerosol and the air passes through the flavor source 131, the flavor component contained in the flavor source 131 is imparted to the aerosol.

The mouthpiece 124 is a member that can be held by the user in his/her mouth during the inhalation. The air outflow hole 182 is disposed in the mouthpiece 124. The user can take in the mixed fluid of the aerosol and the air into an oral cavity by holding the mouthpiece 124 in the mouth and inhaling the mouthpiece 124.

The configuration example of the inhalation device 100A has been described above. It is needless to say that the configuration of the inhalation device 100A is not limited to the above, and may adopt various configurations as exemplified below.

As an example, the inhalation device 100A may not include the flavor imparting cartridge 130. In this case, the cartridge 120 is provided with the mouthpiece 124.

As another example, the inhalation device 100A may include a plurality of types of aerosol sources. A plurality of types of aerosols generated from the plurality of types of aerosol sources may be mixed in the air flow path 180 to cause a chemical reaction, thereby generating another type of aerosol.

A method for atomizing the aerosol source is not limited to heating by the heating portion 121A. For example, the method for atomizing the aerosol source may be vibratory atomization or induction heating.

### (1.2) Second Configuration Example of Inhalation Device

FIG. 1B is a schematic diagram schematically illustrating a second configuration example of the inhalation device. In the inhalation device 100B, for example, a stick-type substrate 150 having a flavor generating substrate such as a filler containing an aerosol source and a flavor source which are inhalation component sources is inserted. In this configuration example, the aerosol source is not limited to a liquid, and may be a solid. The inserted stick-type substrate 150 generates an aerosol containing a flavor component by being heated from an outer periphery thereof.

As illustrated in FIG. 1B, the inhalation device 100B according to this configuration example includes a power supply unit 110B. The power supply unit 110B can be referred to as a main body portion of the inhalation device 100B, and includes a power supply portion 111B, a sensor unit 112B, a notification unit 113B, a storage unit 114B, a communication unit 115B, a power feeding unit 119B, a control unit 116B, a heating portion 121B, a holding portion 140, and a heat insulation portion 144.

The power supply portion 111B, the sensor unit 112B, the notification unit 113B, the storage unit 114B, the communication unit 115B, the power feeding unit 119B, and the control unit 116B are substantially the same as the corresponding components provided in the inhalation device 100A according to the first configuration example.

The holding portion 140 has an internal space 141, and holds the stick-type substrate 150 while accommodating a part of the stick-type substrate 150 in the internal space 141. The holding portion 140 has an opening 142 through which the internal space 141 communicates with the outside, and holds the stick-type substrate 150 inserted into the internal space 141 from the opening 142. For example, the holding portion 140 is a cylindrical body having the opening 142 and a bottom portion 143 as a bottom surface, and defines the columnar internal space 141. In the present specification, a direction in which the stick-type substrate 150 is inserted into a substrate portion 151 is defined as a longitudinal direction of the inhalation device 100B.

The holding portion 140 includes a shutter (not illustrated) that opens and closes the opening 142. More specifically, the shutter includes a slide mechanism and is movable along a surface of an outer shell between a first position where the opening 142 is closed and a second position where the opening 142 is opened. The stick-type substrate 150 is inserted into the substrate portion 151 through the opening 142 in a state where the opening 142 is opened, and is received in the internal space 141. Opening and closing of the opening 142 can be detected by the sensor unit 112B by providing a sensor (not illustrated) in the vicinity of the first position and/or the second position. For example, a magnet is disposed in the shutter, and the opening and closing of the opening 142 is detected by a magnetic sensor.

The communication unit 113B may activate a communication function in response to the opening of the opening 142 of the shutter and start communication with an external device (for example, the panel 10). Alternatively, the communication with the external device in communication may end in response to the closing of the opening 142 of the shutter. In this way, communication with the external device (for example, the panel 10) can be performed in an inhalation period in which the aerosol is inhaled by the user.

In the holding portion 140, a pressing portion and a non-pressing portion (both not illustrated) are formed on an inner wall of the internal space 141 along the longitudinal direction. When the internal space 141 receives the stick-type substrate 150, the pressing portion presses the stick-type substrate 150 in a direction perpendicular to the longitudinal direction. The stick-type substrate 150 is sandwiched by the holding portion 140 while being pressed and deformed by the pressing portion. As a result, the stick-type substrate 150 is heated from the outer periphery thereof by the heating portion 121B while being pressed.

On the other hand, a gap (not illustrated) is formed between the non-pressing portion and the stick-type substrate 150. Accordingly, the opening 142 and the bottom portion 143 communicate with each other through the gap.

The holding portion 140 also has a function of defining a flow path of air fed to the stick-type substrate 150. An air inflow hole 191 which is an inlet of air to the flow path is the opening 142. More precisely, the air inflow hole 191 is a gap between the non-pressing portion and the stick-type substrate 150. Air flowing in from the air inflow hole 191 along with the inhalation by the user is transported to an air outflow hole 192 which is an outlet of air from the flow path through the stick-type substrate 150 along an arrow 190B indicated by a dotted line.

The stick-type substrate 150 includes a substrate portion 151 and an inhalation port portion 152. The substrate portion 151 includes the aerosol source. In a state in which the stick-type substrate 150 is held by the holding portion 140, at least a part of the substrate portion 151 is accommodated in the internal space 141, and at least a part of the inhalation port portion 152 protrudes from the opening 142. Then, when the user holds the inhalation port portion 152 protruding from the opening 142 in the mouth and inhales, the air flows into the internal space 141 from the air inflow hole 191, is transported to the air outflow hole 192 of the inhalation port portion 152 via the bottom portion 143 along the arrow 190B indicated by the dotted line, and reaches an oral cavity of the user together with the aerosol generated from the substrate portion 151.

The heating portion 121B has a configuration similar to that of the heating portion 121A according to the first configuration example. However, in the example illustrated in FIG. 1B, the heating portion 121B is formed in a film shape and disposed to cover an outer periphery of the holding portion 140. Then, when the heating portion 121B generates heat, the substrate portion 151 of the stick-type substrate 150 is heated from the outer periphery thereof, and the aerosol is generated.

The heat insulation portion 144 prevents heat transfer from the heating portion 121B to other components. For example, the heat insulation portion 144 is made of a vacuum heat insulating material or an aerogel heat insulating material.

The configuration example of the inhalation device 100B has been described above. It is needless to say that the configuration of the inhalation device 100B is not limited to the above, and may adopt various configurations as exemplified below.

As an example, the heating portion 121B may be formed in a blade shape and disposed to protrude from the bottom portion 143 of the holding portion 140 into the internal space 141. In this case, the blade-shaped heating portion 121B is inserted into the substrate portion 151 of the stick-type substrate 150, and heats the substrate portion 151 of the stick-type substrate 150 from the inside. As another example, the heating portion 121B may be disposed to cover the bottom portion 143 of the holding portion 140. Further, the heating portion 121B may be implemented by a combination of two or more of a first heating portion covering an outer periphery of the holding portion 140, a blade-shaped second heating portion, and a third heating portion covering the bottom portion 143 of the holding portion 140.

The method for atomizing the aerosol source is not limited to heating by the heating portion 121B. For example, the method for atomizing the aerosol source may be induction heating.

The inhalation device 100B may further include the heating portion 121A, the liquid guide portion 122, the liquid storage portion 123, and the air flow path 180 according to the first configuration example, and the air outflow hole 182 of the air flow path 180 may also serve as the air inflow hole to the internal space 141. In this case, the mixed fluid of the aerosol and the air generated by the heating portion 121A flows into the internal space 141, is further mixed with the aerosol generated by the heating portion 121B, and reaches the oral cavity of the user.

### «2. Configuration Example of Panel»

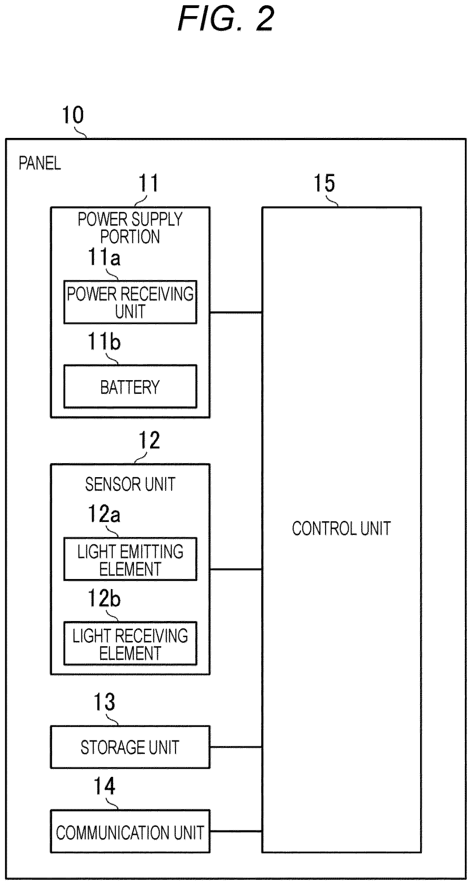

Next, a configuration example of the panel 10 provided in the inhalation device 100 (100A, 100B) according to an embodiment will be described with reference to FIG. 2. Here, the panel 10 mainly includes a member forming at least a part of the outermost housing of the inhalation device 100. The panel 10 is configured to be attachable to and detachable from the power supply unit 110 (110A, 110B) which is a main body portion of the inhalation device 100. In other words, the panel 10 is replaceable.

By making the panel 10 replaceable, the user can easily replace a sensor unit 12 (to be described later) provided on the panel 10 through replacement of the panel 10. Accordingly, for example, even if the sensor unit 12 fails, the user can re-use the function of the inhalation device 100 implemented by using the sensor unit 12 by replacing the panel 10 by himself/herself. Accordingly, even if the sensor unit 12 fails, the inhalation device 100 does not need to be repaired by a manufacturer or the like, and thus the convenience for the user is improved.

Further, by making the panel 10 replaceable, it is possible to change an appearance of the inhalation device 100 through the replacement of the panel 10, and to change the function of the inhalation device 100 implemented by using the panel 10 (for example, the sensor unit 12). Therefore, the user can customize the appearance and function of the inhalation device 100 in accordance with, for example, his/her preference. Accordingly, merchantability of the inhalation device 100 can be improved.

FIG. 2 is a schematic diagram schematically illustrating the configuration example of the panel. As illustrated in FIG. 2, the panel 10 includes a power supply portion 11, the sensor unit 12, a storage unit 13, a communication unit 14, and a control unit 15.

The power supply portion 11 feeds electric power to each component of the panel 10 under the control of the control unit 15. The power supply portion 11 includes, for example, a power receiving unit 11a. The power receiving unit 11a receives the electric power fed from the power supply unit 110 to the panel 10 (in other words, the electric power fed from the power feeding units 119A, 119B to the panel 10). The power supply portion 11 feeds the electric power received by the power receiving unit 11a to each component of the panel 10. Accordingly, each component of the panel 10 including the sensor unit 12 can be operated by the electric power fed from the power supply unit 110 to the panel 10.

As described above, the power supply unit 110 (power feeding units 119A, 119B) may feed the electric power to the panel 10 by the non-contact electric power transmission such as the near field communication. When the power supply unit 110 feeds the electric power to the panel 10 by the near field communication, the power receiving unit 11a may include, for example, the NFC reader and writer module and the NFC antenna.

The power supply portion 11 may further include, for example, a battery 11b that stores the electric power, and may be configured to feed the electric power of the battery 11b to each component of the panel 10. The battery 11b is, for example, a rechargeable battery such as a lithium-ion secondary battery. Further, the battery 11b may be charged with the electric power fed from the power supply unit 110 to the panel 10. As described above, by providing the battery 11b in the panel 10, even if the electric power feeding from the power supply unit 110 to the panel 10 becomes unstable due to some factors, stable electric power can be fed from the battery 11b to each component of the panel 10, and thus the operation can be stabilized.

Further, when the electric power is fed from the power supply unit 110 to the panel 10 by the non-contact electric power transmission such as the near field communication, an upper limit value of the electric power that can be fed to the panel 10 per unit time may be reduced to a certain extent. Thus, the components that can be mounted on the panel 10 (for example, the sensor of the sensor unit 12) can be limited to a sensor with low electric power consumption. In this regard, if the battery 11b is provided in the panel 10, it is possible to provide, in the panel 10, a component that consumes more electric power than the electric power that can be fed from the power supply unit 110 to the panel 10 in real time, and it is possible to improve a degree of freedom of components that can be mounted on the panel 10.

The battery 11b is preferably a rechargeable battery formed in a film shape. By making the battery 11b thin, it is possible to provide the battery 11b in the panel 10 while suppressing an increase in the thickness of the panel 10 (that is, an increase in the size of the inhalation device 100).

The sensor unit 12 is a sensor that obtains information related to the user. More specifically, the sensor unit 12 includes an optical sensor including a light emitting element 12a that emits light to a human body of the user and a light receiving element 12b that receives the light emitted by the light emitting element 12a via the human body of the user and outputting information related to the light received by the light receiving element 12b. The light emitting element 12a is a light source and is implemented by, for example, an LED. The light receiving element 12b is implemented by, for example, a photodiode.

The light received by the light receiving element 12b is, for example, reflected light from the human body. The reflected light includes light scattered and reflected in the human body (that is, scattered light). The light received by the light receiving element 12b may be transmitted light transmitted through the human body.

The output of the sensor unit 12 including the information related to the light received by the light receiving element 12b is, for example, sent to the control unit 15 and then sent from the control unit 15 to the control unit 116 of the power supply unit 110 via the communication unit 14, thereby being provided for the control of the inhalation device 100 by the control unit 116 (to be described later). Here, the information related to the light received by the light receiving element 12b may be, for example, information indicating an intensity, a wavelength, or the like of the light received by the light receiving element 12b.

The sensor unit 12 may include another sensor in addition to the above-described optical sensor. For example, the sensor unit 12 may include a biosensor that obtains predetermined biological information such as a body temperature, a pulse rate, or a perspiration amount of the user. Further, the sensor unit 12 may include an air pressure sensor capable of detecting an outside air pressure (for example, atmospheric pressure) of the inhalation device 100, an air temperature sensor capable of detecting an outside air temperature (for example, room temperature) of the inhalation device 100, an acceleration sensor capable of detecting an acceleration generated in the inhalation device 100, a pressure sensor capable of detecting a pressure applied to the inhalation device 100 from the outside, or the like. As another example, the sensor unit 12 may include a touch sensor capable of detecting contact of the user with the inhalation device 100, a distance sensor capable of detecting a distance between the inhalation device 100 and an object, a color sensor capable of detecting a color of the object, a proximity sensor capable of detecting proximity of the object to the inhalation device 100, an orientation sensor capable of detecting north as a direction, a biometric authentication sensor capable of recognizing a fingerprint, an iris, or the like of the user.

As described above, when the panel 10 is provided with the battery 11b, the sensor unit 12 may further include a battery sensor that detects an output voltage or an input and output current of the battery 11b. By controlling the charging of the battery 11b based on a detection result of such a battery sensor, the battery 11b can be charged appropriately.

For example, the type of the sensor provided in the sensor unit 12 may be different for each type of the panel 10. A function corresponding to the sensor provided in the sensor unit 12 of the panel 10 mounted on the power supply unit 110 may be provided for the user. In this way, the user can change the function provided by the inhalation device 100 through the replacement of the panel 10, and customize the inhalation device 100 such that a function suitable for his/her preference is provided. Accordingly, the convenience and merchantability of the inhalation device 100 are improved.

The storage unit 13 stores various types of information related to the panel 10. The storage unit 13 is implemented by, for example, a nonvolatile storage medium such as a flash memory. For example, the storage unit 13 may store information received by the control unit 15 from the sensor unit 12.

The communication unit 14 is a communication interface that performs communication with the power supply unit 110 (the communication unit 115A or the communication unit 115B) under the control of the control unit 15. The communication unit 14 communicates with the power supply unit 110 by, for example, the near field communication. As described above, communication and electric power transmission between the power supply unit 110 and the panel 10 is efficiently enabled and the configurations of the power supply unit 110 and the panel 10 can be simplified by feeding the electric power from the power supply unit 110 to the panel 10 by the near field communication and the communication unit 14 communicating with the power supply unit 110 by the near field communication. In a case where the electric power feeding from the power supply unit 110 to the panel 10 is performed by the near field communication and the communication unit 14 communicates with the power supply unit 110 by the near field communication, the communication unit 14 can be implemented by the same NFC reader and writer module and NFC antenna as the power receiving unit 11a.

The communication between the communication unit 14 and the power supply unit 110 may be performed using, for example, Wi-Fi (registered trademark) or Bluetooth (registered trademark) without being limited to the near field communication. As described above, when the power supply unit 110 and the panel 10 are connected via a physical power feeding interface, the communication unit 14 may communicate with the power supply unit 110 via the power feeding interface.

The control unit 15 functions as an arithmetic processing device and a control device, and controls overall operations in the panel 10 according to various programs. The control unit 15 is implemented by, for example, an electronic circuit such as a central processing unit (CPU) or a microprocessor.

### <<3. Appearance Configuration Example of Inhalation Device>>

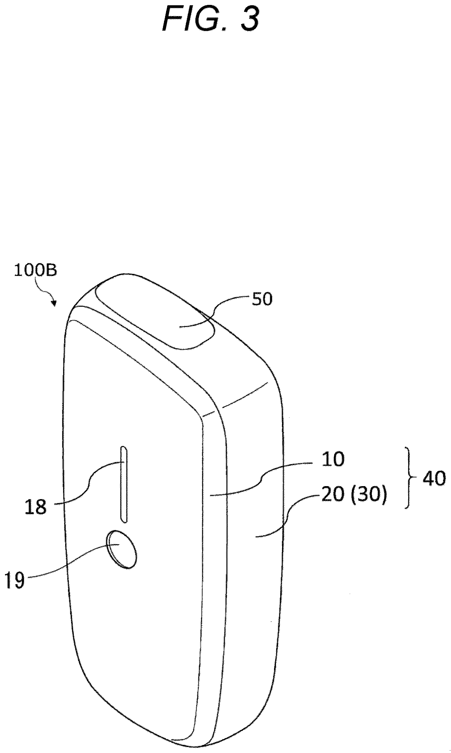

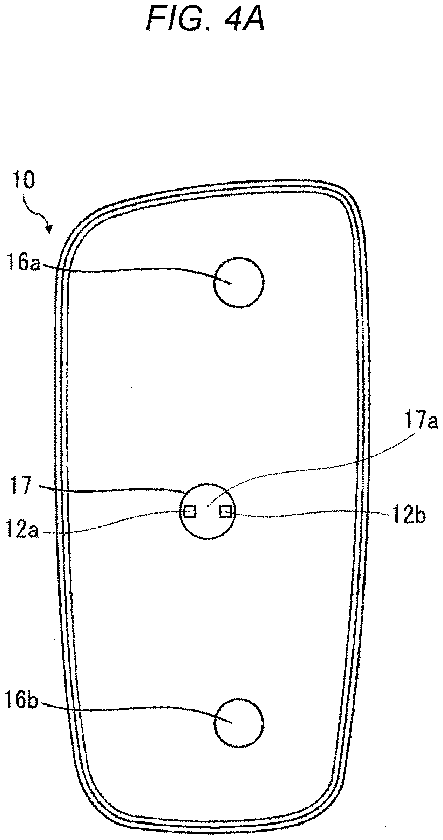

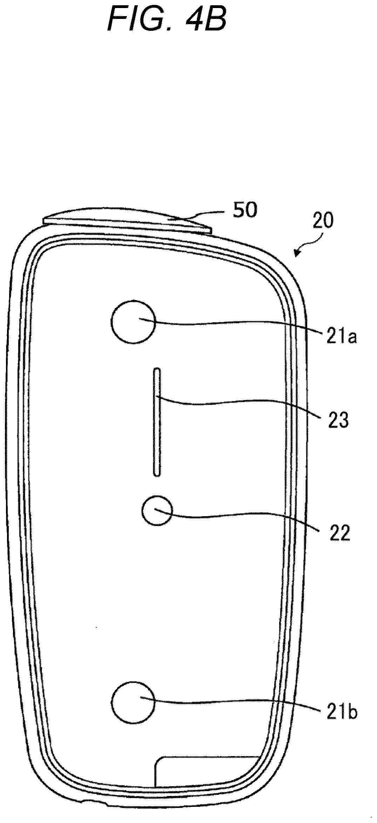

Next, a specific appearance configuration example of the inhalation device 100 according to an embodiment will be described with reference to FIGs. 3, 4A, and 4B. In the following description of the appearance configuration example, in the following description of the appearance configuration example, the inhalation device 100 is described as the inhalation device 100B illustrated in FIG. 1B, but the present invention is not limited thereto, and the same applies to a case where the inhalation device 100 is the inhalation device 100A illustrated in FIG. 1A.

FIG. 3 is an overall perspective view of the inhalation device 100B. The inhalation device 100B includes a panel 10, a main body housing 20 to which and from which the panel 10 is attachable and detachable, and a shutter 50. The panel 10 and the main body housing 20 are formed as separate members. The panel 10 includes a display unit 18 and an operation unit 19 made of a transparent material on a surface (outer surface) thereof.

For example, the operation unit 19 is configured to form a recess toward the main body housing 20. Accordingly, a position of the operation unit 19 can be guided to the user. Further, for example, the position of the operation unit 19 may be guided to the user by printing a predetermined mark (indication) on the surface of the panel 10.

the main body housing 20 accommodates a main body 30 of the inhalation device 100B. Components of the inhalation device 100B illustrated in FIG. 1B are accommodated in the main body 30. the main body housing 20 and the main body 30 can constitute the power supply unit 110B described above.

The panel 10 is attached to the main body housing 20 to form the outermost housing 40 of the inhalation device 100B. For example, fashionability of the inhalation device 100B can be improved by attaching the panel 10 having a design suitable for the preference of the user. Further, since the inhalation device 100B includes the panel 10, even when the main body 30 generates heat, the heat released to the outside can be buffered. That is, the panel 10 functions to insulate heat generated from the heating portion 121B. Further, the panel 10 is formed such that the surface thereof is a substantially curved surface. When attached to the main body housing 20, the panel 10 defines an internal space together with a surface of the main body housing 20.

The housing 40 is preferably sized to be held in a hand of the user. The user holds the inhalation device 100B with one hand while bringing a fingertip into contact with the surface of the panel 10. Further, when the user presses the operation unit 19 with the fingertip, the panel 10 is deformed such that the operation unit 19 is further recessed toward the main body housing 20. As a result of such deformation of the panel 10, a bottom portion of the operation unit 19 comes into contact with an operation button provided on the surface of the main body housing 20, and the operation button is pressed (to be described later).

FIG. 3 shows that the shutter 50 closes the opening 142. The opening 142 is opened when the user slides the shutter 50 along a side surface with a finger. As a result of opening the opening 142, the user can insert the stick-type substrate 150. After inserting the stick-type substrate 150, the user can turn on the power supply of the inhalation device 100B by pressing the operation button by pressing the operation unit 19 with the finger.

### (3.1) Appearance Configuration Example of Panel

FIG. 4A is an external view of an inner surface of the panel 10. FIG. 4B is an external view of an outer surface of the main body housing 20. In a state in which the panel 10 is attached to the main body housing 20, the inner surface of the panel 10 illustrated in FIG. 4A and the outer surface of the main body housing 20 illustrated in FIG. 4B face each other.

As illustrated in FIG. 4A, a magnet 16a, a magnet 16b, and a protrusion 17 are provided on the inner surface of the panel 10. The magnet 16a and the magnet 16b attract the panel 10 to the main body housing 20 by magnetic force (magnetic attraction). Accordingly, the panel 10 is held by the main body housing 20.

The protrusion 17 is a portion corresponding to the operation unit 19 described above on the inner surface of the panel 10. That is, an upper surface portion 17a of the protrusion 17 corresponds to the bottom portion of the operation unit 19 described above. For example, when the sensor unit 12 is a reflective optical sensor, the light emitting element 12a of the sensor unit 12 is provided on the upper surface portion 17a in a state in which a light emitting unit faces the outer surface of the panel 10. The light receiving element 12b of the sensor unit 12 is provided on the upper surface portion 17a in a state in which a light receiving unit faces the outer surface of the panel 10.

A panel circuit unit (not illustrated) is provided on the inner surface of the panel 10. The panel circuit unit is implemented by, for example, an electronic circuit provided with various electronic components that implement the power supply portion 11, the sensor unit 12, the storage unit 13, the communication unit 14, and the control unit 15 described above. The panel circuit unit is connected to the sensor unit 12 (the light emitting element 12a and the light receiving element 12b) by, for example, flexible printed circuits (FPC) (not illustrated).

### (3.2) Appearance Configuration Example of Main Body Housing 20

As illustrated in FIG. 4B, a magnet 21a, a magnet 21b, an operation button 22, and a display window 23 are provided on the outer surface of the main body housing 20. The magnet 21a, the magnet 21b, and the operation button 22 of the main body housing 20 correspond to the magnet 16a, the magnet 16b, and the protrusion 17 of the panel 10, respectively. That is, the panel 10 is aligned with and faces the main body housing 20 when attached to the main body housing 20.

The magnet 21a and the magnet 21b are attracted to the magnet 16a and the magnet 16b of the panel 10 by the magnetic force (magnetic attraction), respectively. That is, the panel 10 is attached to the main body housing 20 by the magnet 16a and the magnet 21a and the magnet 16b and the magnet 21b attracting each other. The magnet 16a and the magnet 16b of the panel 10, and the magnet 21a and the magnet 21b of the main body housing 20 are preferably made of permanent magnets.

The operation button 22 is provided on the surface to which the panel 10 is attached. That is, the operation button 22 is covered with the panel 10 when the panel 10 is attached to the main body housing 20. The display window 23 is an opening aligned with one or more LEDs disposed in the main body 30, and transmits light from the LEDs to the display unit 18 of the panel 10. Accordingly, the user can visually recognize the light from the outer surface of the panel 10. The LED is implemented by the notification unit 113B and issues predetermined notification. For example, the LED issues notification of operation information of the inhalation device 100B in a predetermined light emitting mode. Specifically, the LED emits light to present, to the user, a state indicating whether the inhalation device 100B is powered on, a progress state of preheating, an inhalation state (a remaining inhalable time, and the like), an operation mode (for example, the inhalation mode) in which the inhalation device 100B is currently in, and the like. As will be described later, when the heating of the aerosol source by a heating portion 121 is restricted, the LED may be turned on or blinked in a predetermined light emitting mode to notify the user that the heating of the aerosol source by the heating portion 121 is restricted.

Further, an electronic component (not illustrated) that implements the above-described power feeding unit 119 may be provided on the outer surface of the main body housing 20. Alternatively, the electronic component (for example, an NFC antenna) that implements the power feeding unit 119 may be provided inside the main body housing 20, and a power feeding region for performing the non-contact electric power transmission to the panel 10 may be formed by the electronic component. The power feeding region may also serve as a communication region in which the communication unit 115 can perform communication. Further, a sensor or the like for detecting attachment of the panel 10 to the main body housing 20 may be provided on the outer surface of the main body housing 20.

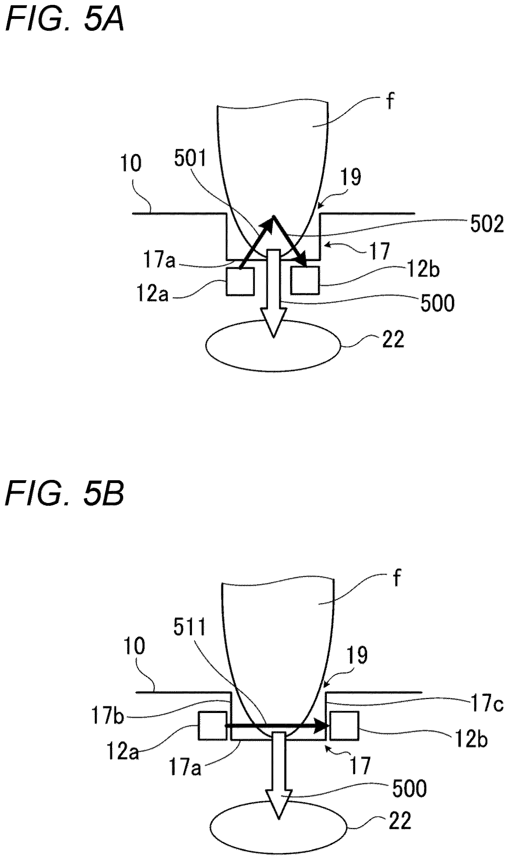

According to the inhalation device 100B configured as described above, as illustrated in FIG. 5A, when the power supply of the inhalation device 100 is turned on, the user presses the operation button 22 by pressing the operation unit 19 with a fingertip f to deform the panel 10 such that the panel 10 is recessed toward the main body housing 20 (see a white arrow indicated by reference numeral 500 in FIG. 5A). When the operation button 22 is pressed in this manner, the control unit 116B of the power supply unit 110B notifies the control unit 15 of the panel 10 via the communication unit 115B that the operation button 22 is pressed, for example.

The control unit 15 of the panel 10 activates the sensor unit 12 in response to the pressing of the operation button 22. Accordingly, the light emitting element 12a of the sensor unit 12 irradiates the fingertip f with light as indicated by an arrow 501. As indicated by an arrow 502, the light receiving element 12b of the sensor unit 12 receives reflected light (including scattered light) from the fingertip f of the light emitted from the light emitting element 12a.

By providing the light emitting element 12a and the light receiving element 12b at positions corresponding to the operation unit 19 in this way, the user can simultaneously perform an operation of turning on the power supply of the inhalation device 100B (in other words, an operation of starting generation of the aerosol) and an operation of causing the sensor unit 12 to obtain the information related to the user. Accordingly, the number of operations by the user can be reduced compared with a case where these operations are separately performed, and the convenience for the user can be improved.

In the example described above, the light emitting element 12a and the light receiving element 12b are provided on the upper surface portion 17a of the protrusion 17 corresponding to the bottom portion of the operation unit 19. For example, when the sensor unit 12 is a transmissive optical sensor, as illustrated in FIG. 5B, the light emitting element 12a and the light receiving element 12b may be provided on wall portions 17b, 17c on both sides of the protrusion 17 so as to face each other. In this case, the light emitting element 12a of the sensor unit 12 irradiates the fingertip f with light as indicated by an arrow 511 in response to pressing of the operation button 22. The light receiving element 12b of the sensor unit 12 receives light (that is, transmitted light) transmitted through the fingertip f.

### «4. Operation Example of Inhalation Device»

Next, an operation example of the inhalation device 100 (100A, 100B) according to an embodiment will be described. The inhalation device 100 (100A, 100B) operates under the control of the control unit 116 (116A, 116B). The control unit 116 controls the operation of the inhalation device 100 based on the output of the sensor unit 12 that is an optical sensor that obtains the information related to the user. Accordingly, the inhalation device 100 can be appropriately operated according to the state of the user.

For example, the quality of an inhalation experience (smoking experience) that the user can experience by using the inhalation device 100 may depend on a physical condition of the user. Therefore, even if the aerosol source or the flavor source is the same as usual (in other words, even if the aerosol source or the flavor source matches the preference of the user), if the physical condition of the user is bad, the user may not be able to obtain a high-quality inhalation experience. Thus, even though the user is in a state of not being able to obtain the high-quality inhalation experience, generating the aerosol in the inhalation device 100 leads to waste of the aerosol source or the flavor source, which is not preferable.

Here, human blood includes plural types of hemoglobin such as carboxyhemoglobin and oxyhemoglobin. The amount of hemoglobin contained in the blood per unit amount of the user (hereinafter, also simply referred to as "content") varies depending on the physical condition of the user. For example, when the content of predetermined hemoglobin (hereinafter, also referred to as "first hemoglobin", for example, carboxyhemoglobin) is a predetermined value or more, there is a high possibility that the physical condition of the user is bad, in other words, the user cannot obtain the high-quality inhalation experience. In addition, with respect to the carboxyhemoglobin, a relation between the carboxyhemoglobin and smoking behavior is known in the related art (for example, see page 138 of URL "http://www.tohoku-kyoritz.jp/jstc2020/pdf/jstc2020.pdf").

In an embodiment, the control unit 116 determines whether the amount of the first hemoglobin (in other words, the content of the first hemoglobin) contained in the blood of the user is the predetermined value or more based on the output of the sensor unit 12 (that is, the light received by the light receiving element 12b), and restricts the heating of the aerosol source by the heating portion 121 based on a result of the determination. Accordingly, the inhalation device 100 can be appropriately operated according to the content of the first hemoglobin. Hereinafter, a specific control example by the control unit 116 will be described in more detail.

### (4.1) Absorbance Characteristics of Each Hemoglobin

Each hemoglobin has different absorbance characteristics, such as with respect to which wavelength of light the absorbance is high and with respect to which wavelength the absorbance is low. Here, the absorbance indicates how much the intensity of light decreases when the light passes through an object serving as a sample substance, and the greater the value, the lower the intensity of light. Further, it is known in the related art that the absorbance characteristics are different for each hemoglobin (for example, see FIG. 4 of URL "https://www.sysmex.co.jp/products_solutions/library/journal/vol4_nol/bfvlfm000000dq8o-att/vol04_1_09.pdf').

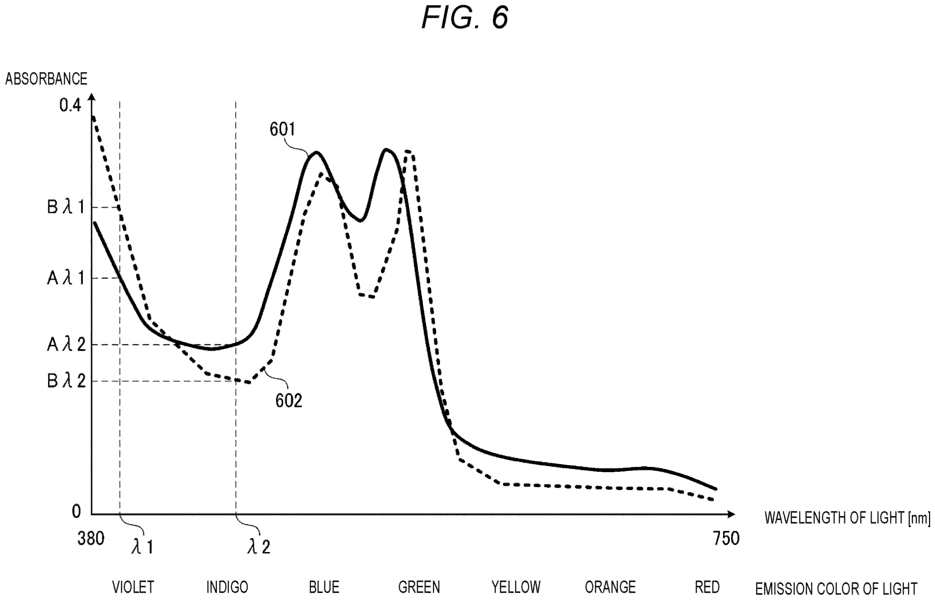

FIG. 6 is a diagram illustrating an example of absorbance characteristics of the first hemoglobin and another hemoglobin (hereinafter, also referred to as "second hemoglobin", for example, oxyhemoglobin) different from the first hemoglobin . In FIG. 6, a vertical axis represents the absorbance, and a horizontal axis represents the wavelength of light.

In FIG. 6, a characteristic 601 indicated by a solid line is an example of the absorbance characteristics of the first hemoglobin. A characteristic 602 indicated by a broken line is an example of the absorbance characteristics of the second hemoglobin. As indicated by the characteristic 601 and the characteristic 602, the absorbance characteristics of the first hemoglobin and the absorbance characteristics of the second hemoglobin are different from each other.

For example, the absorbance of the first hemoglobin with respect to light having a wavelength λ1, which is violet visible light, is Aλ1 (0 < Aλ1). On the other hand, the absorbance of the second hemoglobin with respect to the light having the wavelength λ1 is Bλ1 (Aλ1 < Bλ1). Further, the absorbance of the first hemoglobin with respect to light having a wavelength λ2, which is indigo visible light, is Aλ2 (0 < Aλ2). On the other hand, the absorbance of the second hemoglobin with respect to the light having the wavelength λ2 is Bλ2 (0 < Bλ2 < Aλ2).

Absorbance characteristics of the blood of the user approach the absorbance characteristics of the first hemoglobin as the content of the first hemoglobin increases. Accordingly, in a case where the content of the first hemoglobin is the predetermined value or more, when the light emitting element 12a of the sensor unit 12 irradiates the human body of the user with light, the light received by the light receiving element 12b strongly reflects the absorbance characteristics of the first hemoglobin.

Using such a feature of the light received by the light receiving element 12b, the control unit 116 determines whether the content of the first hemoglobin is the predetermined value or more based on the output of the sensor unit 12. More specifically, first, the control unit 116 derives the absorbance of the blood of the user based on the light emitted by the light emitting element 12a and the output of the sensor unit 12. Information indicating the content (for example, intensity or wavelength) of the light emitted by the light emitting element 12a is stored in advance in the control unit 116, for example. The absorbance of the blood can be derived based on, for example, the intensity of light emitted by the light emitting element 12a and the intensity of light received by the light receiving element 12b.

When a difference between the absorbance of the blood and the absorbance of the first hemoglobin is less than a first threshold, the control unit 116 determines that the content of the first hemoglobin is the predetermined value or more. In other words, when the absorbance of the blood does not deviate from the absorbance of the first hemoglobin by the first threshold or more, the control unit 116 determines that the content of the first hemoglobin is the predetermined value or more.

That is, as described above, the absorbance of the blood approaches the absorbance of the first hemoglobin as the content of the first hemoglobin increases. Using such a relation between the content of the first hemoglobin and the absorbance of the blood, the control unit 116 determines that the content of the first hemoglobin is the predetermined value or more when the difference (that is, a degree of divergence) between the absorbance of the blood and the absorbance of the first hemoglobin is small. Accordingly, it is possible to accurately determine whether the content of the first hemoglobin is the predetermined value or more from the output of the sensor unit 12. Information indicating the absorbance of the first hemoglobin and the first threshold are stored in advance in the control unit 116, for example. The information indicating the absorbance of the first hemoglobin may be appropriately updated or stored by the control unit 116.

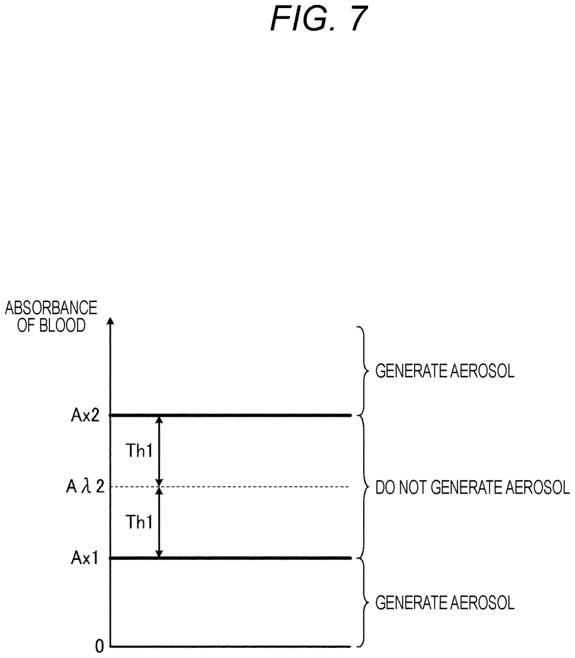

FIG. 7 is a diagram illustrating a first example of control based on an output of the sensor unit by the control unit of the inhalation device according to an embodiment. Here, as an example, it is assumed that the light emitting element 12a irradiates the human body of the user with the light having the wavelength λ2 (that is, the indigo visible light). In this example, the control unit 116 determines that the content of the first hemoglobin is the predetermined value or more when the derived absorbance of the blood does not deviate from Aλ2, which is the absorbance of the first hemoglobin with respect to the light having the wavelength λ2, by a first threshold Th1 or more.

More specifically, as illustrated in FIG. 7, when the absorbance of the blood is greater than Ax1 obtained by subtracting the first threshold Th1 from Aλ2 and smaller than Ax2 obtained by adding the first threshold Th1 to Aλ2, the control unit 116 determines that the content of the first hemoglobin is the predetermined value or more. On the other hand, when the absorbance of the blood is Ax1 or less or Ax2 or more, the control unit 116 determines that the content of the first hemoglobin is the predetermined value or more (in other words, the content of the first hemoglobin is less than the predetermined value).

When determining that the content of the first hemoglobin is the predetermined value or more, the control unit 116 prohibits the heating of the aerosol source by the heating portion 121 by, for example, not feeding electric power to the heating portion 121. Accordingly, no aerosol is generated in this case. Accordingly, it is possible to suppress the occurrence of a situation in which the aerosol source or the flavor source is wasted along with the generation of the aerosol even though the user is in the state of not being able to experience the high-quality inhalation experience.

On the other hand, when determining that the content of the first hemoglobin is less than the predetermined value, the control unit 116 does not prohibit the heating of the aerosol source by the heating portion 121. That is, in this case, the control unit 116 feeds the electric power to the heating portion 121 to heat the heating portion 121, and causes the heating portion 121 to generate aerosol. Accordingly, when the user can experience the high-quality inhalation experience, the user can inhale the aerosol and experience the high-quality inhalation experience, and the user can experience the high-quality inhalation experience.

When determining that the content of the first hemoglobin is the predetermined value or more, the control unit 116 may, for example, prevent the inhalation device 100 from being powered on (in other words, prevent the inhalation device 100 from being activated) even when the above-described operation button 22 is pressed. Even in this case, the generation of the aerosol can be prevented. Accordingly, it is possible to suppress the occurrence of the situation in which the aerosol source or the flavor source is wasted along with the generation of the aerosol even though the user is in the state of not being able to experience the high-quality inhalation experience.

Further, when prohibiting the heating of the aerosol source by the heating portion 121 or prevents the inhalation device 100 from being powered on based on the determination that the content of the first hemoglobin is the predetermined value or more, the control unit 116 may cause a notification unit 113 (113A, 113B) to issue the predetermined notification. As an example, the control unit 116 may notify the user that the heating of the aerosol source by the heating portion 121 is restricted by turning on or blinking the LED disposed in the main body 30 according to a predetermined light emitting mode. Accordingly, even when the heating of the aerosol source by the heating portion 121 is restricted, it is possible to prevent the user from misunderstanding that the inhalation device 100 has failed due to the fact that the aerosol source is not heated.

In the example described here, the light emitting element 12a emits the light having the wavelength λ2. For example, the light emitting element 12a may sequentially emit a plurality of types of light having different wavelengths to the human body of the user. Then, the control unit 116 may derive the absorbance of the blood for each wavelength based on the output of the sensor unit 12 corresponding to each of the plurality of types of light emitted by the light emitting element 12a, and determine whether the content of the first hemoglobin is the predetermined value or more based on the derived absorbance of the blood for each wavelength and the absorbance of the first hemoglobin for each wavelength. In this way, it is possible to more accurately determine whether the content of the first hemoglobin is the predetermined value or more from the output of the sensor unit 12.

Here, although the light emitting element 12a emits the light having the wavelength λ2, which is visible light, the present invention is not limited thereto. For example, the light emitted by the light emitting element 12a may be invisible light such as an ultraviolet ray or an infrared ray. As described above, when the light emitting element 12a sequentially emits the plurality of types of light having different wavelengths, the plurality of types of light may be a combination of visible light and invisible light.

At this time, it is also conceivable that the content of the first hemoglobin increases as the user inhales the aerosol. Therefore, even if the content of the first hemoglobin is less than the predetermined value before the start of the inhalation of the aerosol, a situation in which the content of the first hemoglobin becomes the predetermined value or more along with the subsequent inhalation of the aerosol is also assumed. When such a situation occurs, the user cannot obtain the high-quality inhalation experience after the content of the first hemoglobin becomes the predetermined value or more.

Here, in a case where the difference between the absorbance of the blood and the absorbance of the first hemoglobin is equal to or greater than the first threshold and less than a second threshold that is greater than the first threshold, the control unit 116 may lower a temperature when the aerosol source is heated by the heating portion 121, compared with a case where the difference between the absorbance of the blood and the absorbance of the first hemoglobin is the second threshold or more (hereinafter, also referred to as "normal time"). In other words, in a case where the absorbance of the blood deviates from the absorbance of the first hemoglobin by the first threshold or more and does not deviate from the absorbance of the first hemoglobin by the second threshold or more, the control unit 116 may lower the temperature when the aerosol source is heated by the heating portion 121, compared with a case where the absorbance of the blood deviates from the absorbance of the first hemoglobin by the second threshold or more. The lower the temperature when the aerosol source is heated by the heating portion 121, the smaller the amount of the aerosol to be produced and the amount of the flavor component to be added to the aerosol.

That is, when the difference between the absorbance of the blood and the absorbance of the first hemoglobin is equal to or greater than the first threshold and less than the second threshold, the content of the first hemoglobin is not equal to or greater than the predetermined value before the start of the inhalation of the aerosol (for example, at present), but the content of the first hemoglobin may become equal to or greater than the predetermined value due to the increase in the content of the first hemoglobin along with the inhalation of the aerosol thereafter. Accordingly, in such a case, the control unit 116 reduces the amount of the aerosol to be generated and the amount of the flavor component to be added to the aerosol by lowering the temperature when the aerosol source is heated by the heating portion 121, compared with the normal time. Accordingly, it is possible to suppress an increase in the content of the first hemoglobin along with the inhalation of the aerosol, and it is possible to suppress the occurrence of a situation in which the content of the first hemoglobin is the predetermined value or more.

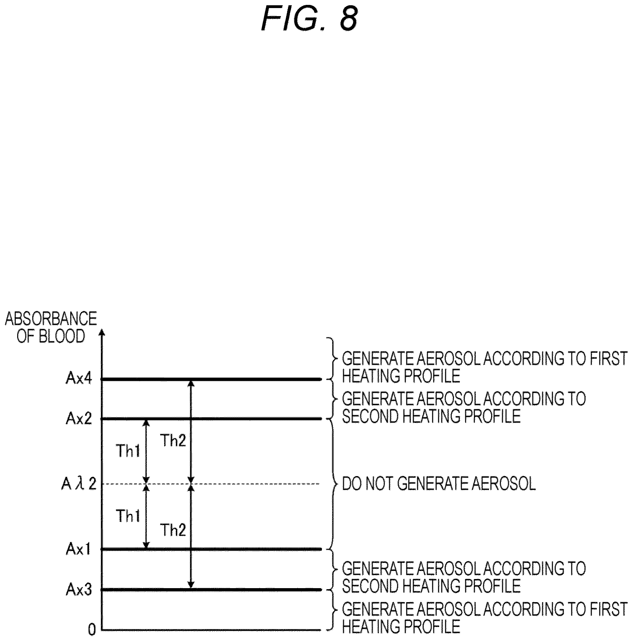

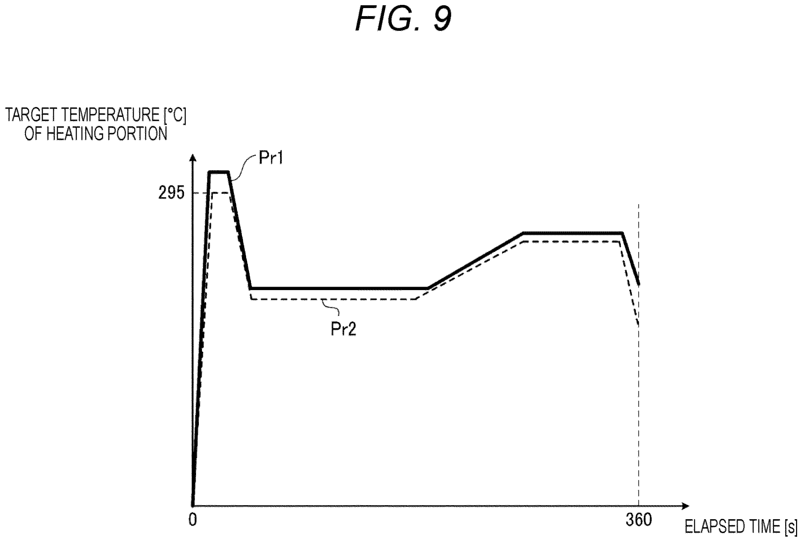

FIG. 8 is a diagram illustrating a second example of the control based on the output of the sensor unit by the control unit of the inhalation device according to an embodiment. FIG. 9 is a diagram illustrating an example of a heating profile of a heating portion in the second example. In the following description of the second example, the description of the same parts as those described in the first example will be appropriately omitted or simplified. In the following description of the second example, the inhalation device 100 is described as the inhalation device 100B illustrated in FIG. 1B, but the present invention is not limited thereto, and the same applies to the case where the inhalation device 100 is the inhalation device 100A illustrated in FIG. 1A.

In this example, when the absorbance of the blood deviates from Aλ2, which is the absorbance of the first hemoglobin with respect to the light having the wavelength λ2, by a second threshold Th2 or more, that is, at the normal time, the control unit 116B heats the heating portion 121B according to the first heating profile Pr1 illustrated in FIG. 9. More specifically, as illustrated in FIG. 8, when the absorbance of the blood is equal to or less than Ax3 obtained by subtracting the second threshold Th2 from Aλ2, or equal to or greater than Ax4 obtained by adding the second threshold Th2 to Aλ2, the control unit 116B heats the heating portion 121B according to the first heating profile Pr1.

On the other hand, when the absorbance of blood deviates from the absorbance of the first hemoglobin by the first threshold Th1 or more and does not deviate from the absorbance of the first hemoglobin by the second threshold Th2 or more, the control unit 116B heats the heating portion 121B according to the second heating profile Pr2 illustrated in FIG. 9. More specifically, as illustrated in FIG. 8, when the absorbance of the blood is greater than Ax3 and equal to or less than Ax1, or is equal to or greater thanAx2 and less than Ax4, the control unit 116B heats the heating portion 121B according to the second heating profile Pr2. Here, as illustrated in FIG. 9, the second heating profile Pr2 is, for example, a heating profile that defines a temperature lower than the first heating profile Pr1 as a target temperature of the heating portion 121B at each timing.

By controlling the temperature of the heating portion 121B according to the second heating profile Pr2, the control unit 116B can reduce the amount of the aerosol to be generated and the amount of the flavor component to be added to the aerosol, compared with the case where the temperature of the heating portion 121B is controlled according to the first heating profile Pr1. Accordingly, it is possible to suppress an increase in the content of the first hemoglobin along with the inhalation of the generated aerosol, and it is possible to suppress an occurrence of a situation in which the content of the first hemoglobin becomes a predetermined value or more along with the inhalation of the generated aerosol.

In the second example, the amount of aerosol to be generated is reduced by lowering the temperature when the aerosol source is heated by the heating portion 121, but the present invention is not limited thereto. For example, the control unit 116 may reduce the amount of aerosol to be generated by reducing the electric power fed to the heating portion 121 when the aerosol is generated.

More specifically, in the case where the difference between the absorbance of the blood and the absorbance of the first hemoglobin is equal to or greater than the first threshold and less than the second threshold that is greater than the first threshold, the control unit 116 may reduce the electric power fed to the heating portion 121 when the aerosol is generated, compared with the case where the difference between the absorbance of the blood and the absorbance of the first hemoglobin is the second threshold or more (that is, normal time). In other words, in a case where the absorbance of the blood deviates from the absorbance of the first hemoglobin by the first threshold or more and does not deviate from the absorbance of the first hemoglobin by the second threshold or more, the control unit 116 may reduce the electric power fed to the heating portion 121 when the aerosol is generated, compared with the case where the absorbance of the blood deviates from the absorbance of the first hemoglobin by the second threshold or more. As a method of reducing the electric power fed to the heating portion 121, for example, a method of shortening feeding time for feeding the electric power to the heating portion 121 and/or a method of lowering a voltage applied to the heating portion 121 when the electric power is fed to the heating portion 121 are considered.

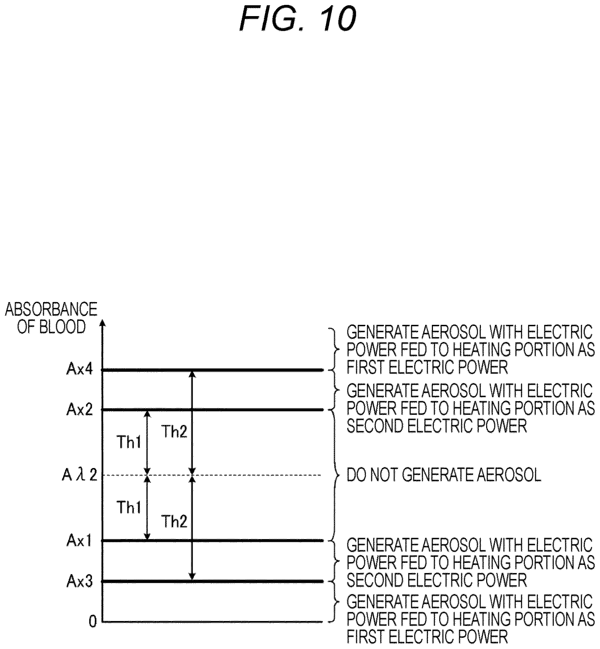

FIG. 10 is a diagram illustrating a third example of the control based on the output of the sensor unit by the control unit of the inhalation device according to an embodiment. In the following description of the third example, the description of the same parts as those described in the first and second examples will be appropriately omitted or simplified. In the following description of the third example, the inhalation device 100 is described as the inhalation device 100A illustrated in FIG. 1A, but the present invention is not limited thereto, and the same applies to the case where the inhalation device 100 is the inhalation device 100B illustrated in FIG. 1B.

In this example, in the case where the absorbance of the blood deviates from Aλ2, which is the absorbance of the first hemoglobin with respect to the light of the wavelength λ2, by the second threshold Th2 or more, that is, at the normal time, the control unit 116A feeds first electric power Pw1 to the heating portion 121A when the aerosol is generated (for example, when the inhalation of the user is detected). More specifically, as illustrated in FIG. 10, when the absorbance of the blood is equal to or less than Ax3 obtained by subtracting the second threshold Th2 from Aλ2, or is equal to or greater than Ax4 obtained by adding the second threshold Th2 to Aλ2, the control unit 116A feeds the first electric power Pw1 to the heating portion 121A when the aerosol is generated.

On the other hand, when the absorbance of the blood deviates from the absorbance of the first hemoglobin by the first threshold Th1 or more and does not deviate from the absorbance of the first hemoglobin by the second threshold Th2 or more, the control unit 116A feeds second electric power Pw2 to the heating portion 121A when the aerosol is generated (specifically, when the inhalation of the user is detected). More specifically, as illustrated in FIG. 10, when the absorbance of the blood is greater than Ax3 and equal to or less thanAx1, or is equal to or greater than Ax2 and less than Ax4, the control unit 116A feeds the second electric power Pw2 to the heating portion 121A when the aerosol is generated.

Here, the second electric power Pw2 is smaller than the first electric power Pw1. For example, when the second electric power Pw2 is fed to the heating portion 121A, the control unit 116A shortens the feeding time for feeding the electric power to the heating portion 121A or lowers the voltage applied to the heating portion 121A, compared with the case where the first electric power Pw1 is fed to the heating portion 121A.

In this way, by setting the electric power fed to the heating portion 121 when the aerosol is generated to the second electric power Pw2, the amount of the aerosol to be generated can be reduced compared with the case where the electric power fed to the heating portion 121 when the aerosol is generated is the first electric power Pw1. Accordingly, it is possible to suppress the increase in the content of the first hemoglobin along with the inhalation of the generated aerosol, and it is possible to suppress the occurrence of the situation in which the content of the first hemoglobin becomes the predetermined value or more along with the inhalation of the generated aerosol.

As described above, according to the inhalation device 100 of an embodiment, since the panel 10 attachable to and detachable from the power supply unit 110 includes the sensor unit 12, the sensor unit 12 can be easily replaced by the user, and convenience can be improved.

Further, according to the inhalation device 100 of an embodiment, it is determined whether the content of the first hemoglobin is the predetermined value or more from the output of the sensor unit 12, and the heating of the aerosol source by the heating portion 121 is restricted based on a result of the determination. Accordingly, the inhalation device 100 can be appropriately operated according to the content of the first hemoglobin.

Further, according to the inhalation device 100 of an embodiment, when it is determined that the content of the first hemoglobin is the predetermined value or more, the heating of the aerosol source by the heating portion 121 is prohibited. Therefore, it is possible to suppress the occurrence of the situation in which the aerosol source is wasted along with the generation of the aerosol even though the user is in the physical condition in which the user cannot experience the high-quality inhalation experience.

Further, according to the inhalation device 100 of an embodiment, the notification unit 113 is caused to issue the predetermined notification when the heating of the aerosol source by the heating portion 121 is restricted. Therefore, even when the heating of the aerosol source by the heating portion 121 is restricted, it is possible to prevent the user from misunderstanding that the inhalation device 100 has failed.

Further, according to the inhalation device 100 of an embodiment, the control unit 116 derives the absorbance of the blood of the user based on the light emitted by the light emitting element 12a and the output of the sensor unit 12 (that is, the light received by the light receiving element 12b), and determines that the content of the first hemoglobin is the predetermined value or more when the difference between the absorbance of the blood and the absorbance of the first hemoglobin is less than the first threshold Th1. Accordingly, it is possible to accurately determine whether the content of the first hemoglobin is the predetermined value or more from the output of the sensor unit 12.

Further, according to the inhalation device 100 of an embodiment, when the difference between the derived absorbance of the blood and the absorbance of the first hemoglobin is equal to or greater than the first threshold Th1 and less than the second threshold Th2 that is greater than the first threshold Th1, the control unit 116 lowers the temperature when the aerosol source is heated by the heating portion 121, compared with when the difference is the second threshold Th2 or more. Accordingly, when there is a possibility that the content of the first hemoglobin becomes the predetermined value or more due to the increase in the first hemoglobin along with the inhalation of the aerosol, the temperature when the aerosol source is heated by the heating portion 121 can be lowered. Therefore, it is possible to suppress the increase in the first hemoglobin along with the inhalation of the aerosol, and it is possible to suppress the occurrence of the situation in which the content of the first hemoglobin becomes the predetermined value or more.

Further, according to the inhalation device 100 of an embodiment, when there is a possibility that the content of the first hemoglobin becomes the predetermined value or more due to the increase in the first hemoglobin along with the inhalation of the aerosol, since the temperature of the heating portion 121 is controlled according to the second heating profile Pr2 in which the target temperature of the heating portion 121 at each timing is lower than the first heating profile Pr1 used at the normal time, it is possible to suppress the increase in the first hemoglobin along with the inhalation of the aerosol, and it is possible to suppress the occurrence of the situation in which the content of the first hemoglobin becomes the predetermined value or more.

Further, according to the inhalation device 100 of an embodiment, when the difference between the derived absorbance of the blood and the absorbance of the first hemoglobin is equal to or greater than the first threshold Th1 and less than the second threshold Th2 that is greater than the first threshold Th1, the control unit 116 reduces the electric power fed to the heating portion 121 when the aerosol is generated compared with when the difference is the second threshold Th2 or more. Accordingly, when there is the possibility that the content of the first hemoglobin becomes the predetermined value or more due to the increase in the predetermined hemoglobin along with the inhalation of the aerosol, it is possible to reduce the electric power fed to the heating portion 121 when the aerosol is generated. Accordingly, it is possible to suppress an increase in the first hemoglobin due to the inhalation of the aerosol, and it is possible to suppress the occurrence of a situation in which the content of the first hemoglobin becomes the predetermined value or more.

Further, according to the inhalation device 100 of an embodiment, when there is the possibility that the content of the first hemoglobin becomes the predetermined value or more due to the increase in the first hemoglobin along with the inhalation of the aerosol, since the feeding time for feeding the electric power to the heating portion 121 is shortened or the voltage applied to the heating portion 121 is lowered compared with the normal time, it is possible to suppress the increase in the first hemoglobin along with the inhalation of the aerosol, and it is possible to suppress the occurrence of the situation in which the content of the first hemoglobin becomes the predetermined value or more.

Further, according to the inhalation device 100 of an embodiment, since the sensor unit 12 is provided on the panel 10 at a position corresponding to the operation unit 19 operated when the user turns on the power supply of the inhalation device 100, the user can simultaneously perform the operation of turning on the power supply of the inhalation device 100 (that is, the operation of starting the generation of the aerosol) and the operation of causing the sensor unit 12 to obtain the information related to the user. Accordingly, the number of operations by the user can be reduced compared with a case where these operations are separately performed, and the convenience can be improved.

Although the embodiments of the present invention have been described above with reference to the along with drawings, it is needless to say that the present invention is not limited to such an embodiment. It is apparent to a person skilled in the art that various changes and modifications may be conceived within the scope described in the claims, and it is understood that the changes and the modifications naturally fall within the technical scope of the present invention. In addition, the constituent components described in the above embodiments may be optionally combined without departing from the spirit of the invention.

For example, in the embodiment described above, the operation unit 19, the light emitting element 12a, and the light receiving element 12b are provided in the panel 10, but the present invention is not limited thereto. For example, the operation unit 19, the light emitting element 12a, and the light receiving element 12b may be provided in the power supply unit 110 (for example, the main body housing 20 or the main body 30) which is the main body portion of the inhalation device 100. That is, the sensor unit 12 including the light emitting element 12a and the light receiving element 12b may be provided in the sensor unit 112 (112A, 112B). Hereinafter, a modification in which the operation unit 19, the light emitting element 12a, and the light receiving element 12b are provided in the power supply unit 110 which is the main body portion of the inhalation device 100 will be described. In the following description of the modification, the description of the same parts as those described in the above-described embodiment will be appropriately omitted or simplified.

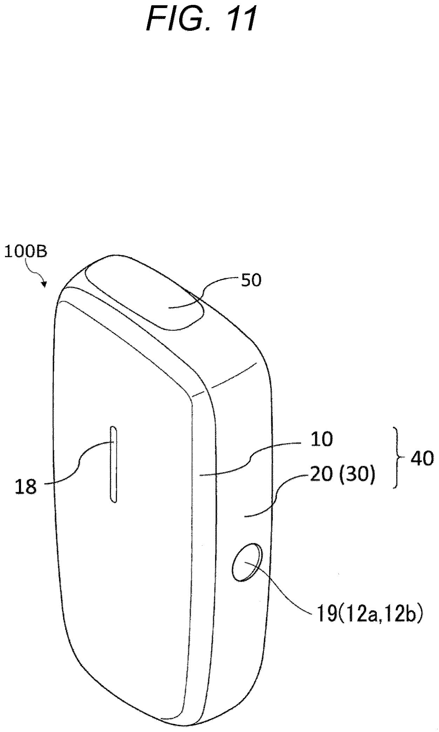

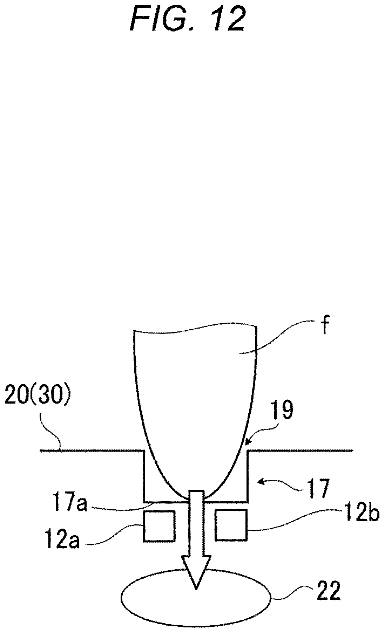

FIG. 11 is an overall perspective view of an inhalation device according to the modification of an embodiment. FIG. 12 is a diagram illustrating an example of a positional relation between an operation unit and a light emitting element and a light receiving element in the inhalation device according to the modification. As illustrated in FIG. 11, in this example, the operation unit 19 is provided on a surface of the main body housing 20 which is not covered with the panel 10. In this example, as illustrated in FIG. 12, the light emitting element 12a, the light receiving element 12b, and the operation button 22 are provided at positions corresponding to the operation unit 19 inside the main body housing 20.

Even in the inhalation device 100 of the modification configured as described above, a user can simultaneously perform an operation of turning on power supply of the inhalation device 100B (in other words, an operation of starting generation of the aerosol) and an operation of causing the sensor unit 12 to obtain information related to the user. Accordingly, the number of operations by the user can be reduced as compared with a case where these operations are separately performed, and convenience for the user can be improved.

Here, the light emitting element 12a and the light receiving element 12b are provided on the upper surface portion 17a of the protrusion 17 corresponding to a bottom portion of the operation unit 19. For example, when the sensor unit 12 is a transmissive optical sensor, similar to the example illustrated in FIG. 5B, the light emitting element 12a and the light receiving element 12b may be provided on the wall portions 17b, 17c on both sides of the protrusion 17 so as to face each other.

Further, the control unit 116 may obtain information indicating the content of first hemoglobin based on the output of the sensor unit 12, and causes the communication unit 115 to transmit health data including the information to an external device (for example, a terminal device of the user). Accordingly, the user can grasp the transition of his/her health state by checking the health data on the terminal device. When the sensor unit 12 includes a biosensor that obtains biological information of the user such as body temperature, a pulse rate, or a perspiration amount of the user, the health data may include the biological information obtained by the biosensor.

Further, when limiting the heating of the aerosol source by the heating portion 121 based on the output of the sensor unit 12, the control unit 116 may cause the communication unit 115 to transmit a signal indicating that the heating of the aerosol source is limited to the external device (for example, the terminal device of a user), and cause the external device to notify that the heating of the aerosol source is limited.

In the present specification, at least the following matters are described. In parentheses, corresponding components and the like in the above-described embodiments are illustrated as an example, and the present invention is not limited thereto.
(1) An inhalation device including a main body portion (power supply units 110A, 110B) and a panel (a panel 10) attachable to and detachable from the main body portion, in which
   the panel includes a sensor (a sensor unit 12),
   the sensor includes a light emitting element (a light emitting element 12a) configured to irradiate a human body of a user with light and a light receiving element (a light receiving element 12b) configured to receive the light via the human body, and outputs information related to the light received by the light receiving element, and
   the main body portion includes a control unit (control units 116A, 116B) configured to control an operation of the inhalation device based on an output of the sensor.
   According to (1), since the panel attachable to and detachable from the main body portion, that is, the replaceable panel includes the sensor, convenience for the user in the inhalation device can be improved.
(2) The inhalation device according to (1), further including:
   a heating portion (heating portions 121A, 121B), in which
   the inhalation device is capable of delivering, to the user, an aerosol generated by heating an aerosol source by the heating portion, and
   the control unit determines whether an amount of predetermined hemoglobin contained in blood of the user is a predetermined value or more based on the output of the sensor, and
   the control unit restricts heating of the aerosol source by the heating portion based on a result of the determination.
   According to (2), it is determined whether the amount of predetermined hemoglobin contained in the blood of the user is the predetermined value or more from the output of the sensor, and the heating of the aerosol source by the heating portion is restricted based on the result of the determination. Accordingly, the inhalation device can be appropriately operated according to the amount of predetermined hemoglobin contained in the blood of the user.
(3) The inhalation device according to (2), in which
   the control unit prohibits the heating of the aerosol source by the heating portion when determining that the amount of the predetermined hemoglobin contained in the blood is the predetermined value or more.
   According to (3), when it is determined that the amount of the predetermined hemoglobin contained in the blood of the user is the predetermined value or more, the heating of the aerosol source by the heating portion is prohibited. Therefore, it is possible to suppress the occurrence of a situation in which the aerosol source is wasted due to the generation of the aerosol even though the user is in physical condition in which the user cannot experience a high-quality inhalation experience.
(4) The inhalation device according to (2) or (3), in which
   the main body portion further includes a notification unit (notification units 113A, 113B) configured to issue predetermined notification to the user, and
   the control unit causes the notification unit to issue the predetermined notification when the heating of the aerosol source by the heating portion is restricted.
   According to (4), the notification unit is caused to issue the predetermined notification when the heating of the aerosol source by the heating portion is restricted. Therefore, even when the heating of the aerosol source by the heating portion is restricted, it is possible to prevent the user from misunderstanding that the inhalation device has failed.
(5) The inhalation device according to any one of (2) to (4), in which
   the control unit derives absorbance of the blood of the user based on the light emitted by the light emitting element and the output of the sensor, and
   the control unit determines that the amount of the predetermined hemoglobin contained in the blood is the predetermined value or more when a difference between the derived absorbance of the blood and absorbance of the predetermined hemoglobin is less than a first threshold (a first threshold Th1).
   According to (5), it is possible to accurately determine whether the amount of predetermined hemoglobin contained in the blood of the user is the predetermined value or more from the output of the sensor.
(6) The inhalation device according to (5), in which
   the control unit lowers a temperature when the aerosol source is heated by the heating portion in a case where the difference is equal to or greater than the first threshold and less than a second threshold that is greater than the first threshold, compared with a case where the difference is the second threshold or more.
   It is also conceivable that as the user inhales the aerosol, the amount of predetermined hemoglobin contained in the blood of the user increases. According to (6), when there is a possibility that the amount of the predetermined hemoglobin contained in the blood of the user becomes the predetermined value or more due to the increase in the predetermined hemoglobin along with the inhalation of the aerosol, the temperature when the aerosol source is heated by the heating portion can be lowered. Accordingly, it is possible to suppress the increase in the predetermined hemoglobin along with the inhalation of the aerosol, and it is possible to suppress the occurrence of a situation in which the amount of the predetermined hemoglobin contained in the blood of the user is the predetermined value or more.
(7) The inhalation device according to (6), in which
   the control unit is configured to control temperature of the heating portion according to a heating profile which is information defining a time-series transition of a target temperature of the heating portion when the heating portion is heated,
   the control unit controls the temperature of the heating portion according to a first heating profile (a first heating profile Pr1), when the difference is the second threshold or more, and
   the control unit controls the temperature of the heating portion according to a second heating profile (a second heating profile Pr2) in which the target temperature of the heating portion at each timing is lower than the first heating profile, when the difference is the first threshold or more and less than the second threshold.
   According to (7), when there is the possibility that the amount of the predetermined hemoglobin contained in the blood of the user becomes the predetermined value or more due to the increase in the predetermined hemoglobin along with the inhalation of the aerosol, since the temperature of the heating portion is controlled according to the second heating profile in which the target temperature of the heating portion at each time is lower than the first heating profile used at the normal time, it is possible to suppress the increase in the predetermined hemoglobin along with the inhalation of the aerosol, and it is possible to suppress the occurrence of the situation in which the amount of the predetermined hemoglobin contained in the blood of the user becomes the predetermined value or more.
(8) The inhalation device according to (5), in which
   the control unit reduces electric power that is fed to the heating portion when the aerosol is generated in a case where the difference is equal to or greater than the first threshold and less than a second threshold that is greater than the first threshold, compared with a case where the difference is the second threshold or more.
   It is also conceivable that as the user inhales the aerosol, the amount of predetermined hemoglobin contained in the blood of the user increases. According to (8), when there is the possibility that the amount of the predetermined hemoglobin contained in the blood of the user becomes the predetermined value or more due to the increase in the predetermined hemoglobin along with the inhalation of the aerosol, the electric power fed to the heating portion when the aerosol is generated can be reduced. Accordingly, it is possible to suppress the increase in the predetermined hemoglobin along with the inhalation of the aerosol, and it is possible to suppress the occurrence of a situation in which the amount of the predetermined hemoglobin contained in the blood of the user is the predetermined value or more.
(9) The inhalation device according to (8), in which
   the control unit shortens feeding time for feeding the electric power to the heating portion or lowers a voltage applied to the heating portion in a case where the difference is equal to or greater than the first threshold and less than the second threshold, compared with the case where the difference is the second threshold or more.
   According to (9), when there is the possibility that the amount of the predetermined hemoglobin contained in the blood of the user becomes the predetermined value or more due to the increase in the predetermined hemoglobin along with the inhalation of the aerosol, since the feeding time for feeding the electric power to the heating portion is shortened or the voltage applied to the heating portion is lowered compared with the normal time, it is possible to suppress the increase in the predetermined hemoglobin along with the inhalation of the aerosol, and it is possible to suppress the occurrence of the situation in which the amount of the predetermined hemoglobin contained in the blood of the user becomes the predetermined value or more.
(10) The inhalation device according to any one of (1) to (9), in which
   the sensor is provided on the panel at a position corresponding to an operation unit (an operation unit 19) operated when the user turns on a power supply of the inhalation device.

According to (10), since the sensor is provided at a position corresponding to the operation unit operated when the user turns on the power supply of the inhalation device, the user can simultaneously perform the operation of turning on the power supply of the inhalation device (that is, an operation to start generation of the aerosol) and an operation to cause the sensor to obtain the information related to the user. Accordingly, the number of operations by the user can be reduced compared with a case where these operations are separately performed, and the convenience can be improved.

### REFERENCE SIGNS LIST

10 panel
12 sensor unit (sensor)
12a light emitting element
12b light receiving element
19 operation unit
100A, 100B inhalation device
110A, 110B power supply unit (main body portion)
113A, 113B notification unit
116A, 116B control unit
121A, 121B heating portion
Pr1 first heating profile
Pr2 second heating profile

## Claims

1. An inhalation device comprising a main body portion and a panel attachable to and detachable from the main body portion, wherein
the panel includes a sensor,
the sensor includes a light emitting element configured to irradiate a human body of a user with light and a light receiving element configured to receive the light via the human body, and outputs information related to the light received by the light receiving element, and
the main body portion includes a control unit configured to control an operation of the inhalation device based on an output of the sensor.

2. The inhalation device according to claim 1, further comprising a heating portion, wherein
the inhalation device is capable of delivering, to the user, an aerosol generated by heating an aerosol source by the heating portion, and
the control unit determines whether an amount of predetermined hemoglobin contained in blood of the user is a predetermined value or more based on the output of the sensor, and
the control unit restricts heating of the aerosol source by the heating portion based on a result of the determination.

3. The inhalation device according to claim 2, wherein
the control unit prohibits the heating of the aerosol source by the heating portion when determining that the amount of the predetermined hemoglobin contained in the blood is the predetermined value or more.

4. The inhalation device according to claim 2 or 3, wherein
the main body portion further includes a notification unit configured to issue predetermined notification to the user, and
the control unit causes the notification unit to issue the predetermined notification when the heating of the aerosol source by the heating portion is restricted.

5. The inhalation device according to any one of claims 2 to 4, wherein
the control unit derives absorbance of the blood of the user based on the light emitted by the light emitting element and the output of the sensor, and
the control unit determines that the amount of the predetermined hemoglobin contained in the blood is the predetermined value or more when a difference between the derived absorbance of the blood and absorbance of the predetermined hemoglobin is less than a first threshold.

6. The inhalation device according to claim 5, wherein
the control unit lowers a temperature when the aerosol source is heated by the heating portion in a case where the difference is equal to or greater than the first threshold and less than a second threshold that is greater than the first threshold, compared with a case where the difference is the second threshold or more.

7. The inhalation device according to claim 6, wherein
the control unit is configured to control temperature of the heating portion according to a heating profile which is information defining a time-series transition of a target temperature of the heating portion when the heating portion is heated,
the control unit controls the temperature of the heating portion according to a first heating profile, when the difference is the second threshold or more, and
the control unit controls the temperature of the heating portion according to a second heating profile in which the target temperature of the heating portion at each timing is lower than the first heating profile, when the difference is the first threshold or more and less than the second threshold.

8. The inhalation device according to claim 5, wherein
the control unit reduces electric power that is fed to the heating portion when the aerosol is generated in a case where the difference is equal to or greater than the first threshold and less than a second threshold that is greater than the first threshold, compared with a case where the difference is the second threshold or more.

9. The inhalation device according to claim 8, wherein
the control unit shortens feeding time for feeding the electric power to the heating portion or lowers a voltage applied to the heating portion in a case where the difference is equal to or greater than the first threshold and less than the second threshold, compared with the case where the difference is the second threshold or more.

10. The inhalation device according to any one of claims 1 to 9, wherein
the sensor is provided on the panel at a position corresponding to an operation unit operated when the user turns on a power supply of the inhalation device.
